(19) Europäisches Patentamt
European Patent Office
Office européen des brevets

(11) EP 0 366 640 B1

(12) EUROPEAN PATENT SPECIFICATION

(45) Date of publication and mention of the grant of the patent:
**03.02.1999 Bulletin 1999/05**

(51) Int. Cl.[6]: **C07D 501/26**, A61K 31/545

(21) Application number: **89870151.1**

(22) Date of filing: **20.10.1989**

# (54) Antimicrobial fluoroquinolonyl cephems

Antimikrobielle Fluorochinolonylcephemderivate

Fluoroquinolonyl céphems antimicrobiens

(84) Designated Contracting States:
**AT BE CH DE ES FR GB GR IT LI LU NL SE**

(30) Priority: **24.10.1988 US 261949**
**12.10.1989 US 418025**

(43) Date of publication of application:
**02.05.1990 Bulletin 1990/18**

(73) Proprietor:
**Procter & Gamble Pharmaceuticals, Inc.**
**Norwich New York 13815 (US)**

(72) Inventors:
- **Demuth, Thomas Prosser, Jr.**
**Norwich New York 13815 (US)**
- **White, Ronald Eugene**
**South Plymouth New York 13844 (US)**

(74) Representative:
**Woof, Victoria et al**
**Patent Dept.,**
**Procter & Gamble Technical Centres Limited,**
**Rusham Park,**
**Whitehall Lane**
**Egham TW20 9NW (GB)**

(56) References cited:
**EP-A- 0 251 330** **EP-A- 0 322 810**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 366 640 B1

## Description

This invention relates to novel antimicrobial compounds and compositions. The compounds of this invention contain a quinolone moiety and a cephem moiety, in a new chemical entity.

The chemical and medical literature describes a myriad of compounds that are said to be antimicrobial, i.e., capable of destroying or suppressing the growth or reproduction of microorganisms, such as bacteria. In particular, antibacterials include a large variety of naturally-occurring (antibiotic), synthetic, or semi-synthetic compounds. They may be classified (for example) as the aminoglycosides, ansamacrolides, beta-lactams (including penicillins and cephalosporins), lincosaminides, macrolides, nitrofurans, nucleosides, oligosaccharides, peptides and polypeptides, phenazines, polyenes, polyethers, quinolones, tetracyclines, and sulfonamides. Such antibacterials and other antimicrobials are described in Antibiotics, Chemotherapeutics, and Antibacterial Agents for Disease Control (M. Grayson, editor, 1982), and E. Gale et al., The Molecular Basis of Antibiotic Action 2d edition (1981).

The mechanism of action of these antibacterials vary. However, each can be generally classified as functioning in one or more of four ways: by inhibiting cell wall synthesis or repair; by altering cell wall permeability; by inhibiting protein synthesis; or by inhibiting synthesis of nucleic acids. For example, beta-lactam antibacterials act through inhibiting the essential penicillin binding proteins (PBPs) in bacteria, which are responsible for cell wall synthesis. On the other hand, quinolones act by inhibiting synthesis of bacterial DNA, thus preventing the bacteria from replicating.

Not surprisingly, the pharmacological characteristics of antibacterials and other antimicrobials, and their suitability for any given clinical use, also vary considerably. For example. the classes of antimicrobials (and members within a class) may vary in their relative efficacy against different types of microorganisms, and their susceptibility to development of microbial resistance. These antimicrobials may also differ in their pharmacological characteristics, such as their bioavailability, and biodistribution. Accordingly, selection of an appropriate antibacterial (or other antimicrobial) in any given clinical situation can be a complicated analysis of many factors, including the type of organism involved, the desired method of administration, and the location of the infection to be treated.

The development of microbial resistance is one factor in the selection of an appropriate antimicrobial (particularly antibacterials), which is of increasing concern in medical science. This "resistance" can be defined as existence of organisms, within a population of a given microbial species, that are less susceptible to the action of a given antimicrobial agent. Such resistant strains may subvert the mechanism of action of a particular antimicrobial, or chemically degrade the antimicrobial before it can act. For example, bacterial resistance to beta-lactam antibacterials has arisen through development of bacterial strains that produce beta-lactamase enzymes, which degrade the antibacterial.

In part as a result of the intense use of antibacterials over extended periods of time, many highly resistant strains of bacteria have evolved. This is of particular concern in environments such as hospitals and nursing homes, which are characterized by relatively high rates of infection and intense use of antibacterials. See, e.g., W. Sanders, Jr. et al., "Inductible Beta-lactamases: Clinical and Epidemiologic Implications for Use of Newer Cephalosporins", 10 Reviews of Infectious Diseases 830 (1988). Indeed, the development of resistant bacterial strains has led to a concern that pathogenic bacteria may be produced that are essentially resistant to even the newest developed antibacterial agents.

The literature describes many attempts to enhance the efficacy of antimicrobials, and to overcome the development of microbial resistance. Many such attempts involve the combination of antimicrobials. For example, Thabaut et al., 16 Presse Med. 2167 (1987) describes combinations of pefloxacin (a quinolone) with the beta-lactams cefotaxime and cefsulodin. Lenoc et al., 36 Path. Biol. 762 (1988), describes combined use of cephems with aminoglycosides, and with quinolones. Japanese Patent Publication 60/06,617, published January 14, 1985, also describes compositions containing beta-lactams and quinolones. O'Callaghan et al., 10 Antimicrobial Agents and Chemotherapy 245 (1976), describes a mercapto pyridine-substituted cephem, which is said to liberate an active antimicrobial agent when the cephalosporin is hydrolyzed by beta-lactamase. Mobashery et al., 108 J. American Chemical Society 1684 (1986), presents a theory of employing bacterial beta-lactamase in situ to release an antibacterially-active leaving group from the 10-position of a cephem.

However, many such attempts to produce improved antimicrobials yield equivocal results. Indeed, few antimicrobials are produced that are truly clinically-acceptable in terms of their spectrum of antimicrobial activity, avoidance of microbial resistance, and pharmacology.

The present invention provides a compound of the formula:

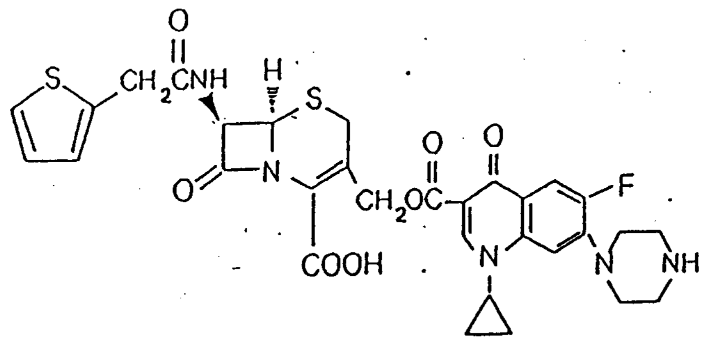

and pharmaceutically-acceptable salts [and biohydrolyzable esters thereof, and hydrates] thereof.

It has been found that the compounds of this invention, and compositions containing these compounds, are effective antimicrobial agents against a broad range of pathogenic microorganisms. These compounds provide advantages versus antimicrobial agents among those known in the art, including (for example) the spectrum of antimicrobial activity, potency, the avoidance of microbial resistance, and reduced toxicity.

The present invention encompasses certain novel fluoroquinolonyl cephems, methods for their manufacture, use; dosage forms, and methods of administering the fluoroquinolonyl cephems to a human or other animal subject. Specific compounds and compositions to be used in the invention must, accordingly, be pharmaceutically acceptable. As used herein, such a "pharmaceutically-acceptable" component is one that is suitable for use with humans and/or animals without undue adverse side effects (such as toxicity, irritation, and allergic response) commensurate with a reasonable benefit/risk ratio.

Fluoroquinolonyl Cephems:

The compound of this invention, herein referred to as "fluoroquinolonyl cephem", is of the formula:

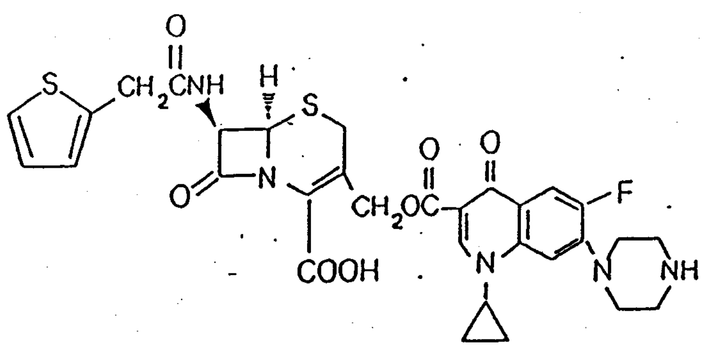

and pharmaceutically-acceptable salts [and biohydrolyzable esters thereof, and hydrates] thereof.

Definitions and Usage of Terms:

The following is a list of definitions for terms used herein.

A "pharmaceutically-acceptable salt" is a cationic salt formed at any acidic (e.g., carboxyl) group, or an anionic salt formed at any basic (e.g., amino) group. Many such salts are known in the art, as described in World Patent Publication 87/05297, Johnston et al., published September 11, 1987. Preferred cationic salts include the alkali metal salts (such as sodium and potassium), and alkaline earth metal salts (such as magnesium and calcium). Preferred anionic salts include the halides (such as chloride salts).

A "biohydrolyzable ester" is an ester of a fluoroquinolonyl cephem that does not essentially interfere with the antimicrobial activity of the compounds, or that are readily metabolized by a human or lower animal subject to yield an antimicrobially-active fluoroquinolonyl cephem. Such esters include those that do not interfere with the biological activity of quinolone antimicrobials or beta-lactam antimicrobials (cephems, for example). Many such esters are known in the art, as described in World Patent Publication 87/05297, Johnston et al., published September 11, 1987. Such esters

include lower alkyl esters, lower acyloxy-alkyl esters (such as acetoxymethyl, acetoxyethyl, aminocarbonyloxymethyl, pivaloyloxymethyl and pivaloyloxyethyl esters), lactonyl esters (such as phthalidyl and thiophthalidyl esters), lower alkoxyacyloxyalkyl esters (such as methoxycarbonyloxymethyl, ethoxycarbonyloxyethyl and isopropoxycarbonyloxyethyl esters), alkoxyalkyl esters, choline esters and alkyl acylamino alkyl esters (such as acetamidomethyl esters).

As defined above and as used herein, substituent groups may themselves be substituted. Such substitution may be with one or more substituents. Such substituents include those listed in C. Hansch and A. Leo, Substituent Constants for Correlation Analysis in Chemistry and Biology (1979). Preferred substituents include (for example) alkyl, alkenyl, alkoxy, hydroxy, oxo, nitro, amino, aminoalkyl (e.g., aminomethyl, etc.), cyano, halo, carboxy, alkoxyaceyl (e.g., carboethoxy, etc.), thiol, aryl, cycloalkyl, heteroaryl, heterocycloalkyl (e.g., piperidinyl, morpholinyl, pyrrolidinyl, etc.), imino, thioxo, hydroxyalkyl, aryloxy, arylalkyl, and combinations thereof.

Methods of Manufacture:

The fluoroquinolonyl cephems of this invention may be made by the following general reaction sequence:

$$\text{Ceph-CH}_2\text{-X} + \text{M}^{+-}\text{OC(=O)-Quin} \rightarrow \text{Ceph-CH}_2\text{-OC(=O)-Quin}$$

where X is a reactive leaving group (such as halo, a sulfonate ester or other activated hydroxyl functionality), "Ceph" generically represents an appropriately protected cephalosporin and "Quin" represents an appropriately Protected quinolone. The reaction can be envisioned as a nucleophilic displacement of the reactive X substituent from the cephalosporin by the quinolone carboxylic acid or salt, to form an ester coupled conjugate of the cephalosporin and quinolone.

For Ceph and Quin, certain functional groups contained in the structures (such as carboxyl, hydroxyl, and amino groups) may need to be blocked in order to prevent undesired, competing side reactions with X. For example, suitable protecting groups for carboxyl substituents include esters; protecting groups for hydroxyl substituents include ethers, esters, and carbonates; and protecting groups for amino substituents include carbamates, amides, and carbonates. If such protecting groups are employed, then appropriate deprotecting chemistry, that will not decompose the ester coupled conjugate, may be required to obtain antimicrobially active products.

Depending on the $R^1$ group desired, the cephalosporin starting material may be available from any of a variety of commercial sources. Synthetic methods for producing such beta-lactams are well-known in the chemical literature. See, for example, Antibiotics, Chemotherapeutics, and Antibacterial Agents for Disease Control, pages 107-125 (M. Grayson, editor, 1982). The cephalosporin starting material can be esterified and converted to the 10-iodo derivative by methods well-known in the art. The fluoroquinolone starting material may also be commercially available (e.g., 1-cyclopropyl-6-fluoro-1,4-dihydro-4-oxo-7-(1-piperazinyl)-3-quinolinecarboxylic acid, known as "ciprofloxacin"). Syntheses of fluoroquinolones are described in US-A-4,670,444, Grohe et al., issued June 2, 1987. The quinolone starting material may be made with appropriate protecting groups, by methods well-known in the art. For example, the piperazine nitrogen of ciprofloxacin can be readily converted to an alkyl carbamate.

Compositions:

The compositions of this invention comprise:

(a) a safe and effective amount of a fluoroquinolonyl cephem of the invention; and
(b) a pharmaceutically-acceptable carrier.

A "safe and effective amount" of a fluoroquinolonyl cephem is an amount that is effective, to inhibit microbial growth at the site of an infection to be treated in a human or lower animal subject, without undue adverse side effects (such as toxicity, irritation, or allergic response), commensurate with a reasonable benefit/risk ratio when used in the manner of this invention. The specific "safe and effective amount" will, obviously, vary with such factors as the particular condition being treated, the physical condition of the patient, the duration of treatment, the nature of concurrent therapy (if any), the specific dosage form to be used, the carrier employed, the solubility of the fluoroquinolonyl cephem therein, and the dosage regimen desired for the composition.

The compositions of this invention are preferably provided in unit dosage form. As used herein, a "unit dosage form" is a composition of this invention containing an amount of a fluoroquinolonyl cephem that is suitable for administration to a human or lower animal subject, in a single dose, according to good medical practice. These compositions preferably contain from about 30 mg to about 20,000 mg, more preferably from about 50 mg (milligrams) to about 7000 mg, more preferably from about 500 mg to about 1500 mg, of a fluoroquinolonyl cephem.

The compositions of this invention may be in any of a variety of forms, suitable (for example) for oral, rectal,

topical or parenteral administration. Depending upon the particular route of administration desired, a variety of pharmaceutically-acceptable carriers well-known in the art may be used. These include solid or liquid fillers, diluents, hydrotropes, surface-active agents, and encapsulating substances. Optional pharmaceutically-active materials may be included, which do not substantially interfere with the antimicrobial activity of the fluoroquinolonyl cephem. The amount of carrier employed in conjunction with the fluoroquinolonyl cephem is sufficient to provide a practical quantity of material for administration per unit dose of the fluoroquinolonyl cephem. Techniques and compositions for making dosage forms useful in the methods of this invention are described in the following references: 7 Modern Pharmaceutics, Chapters 9 and 10 (Banker & Rhodes, editors, 1979); Lieberman et al., Pharmaceutical Dosage Forms: Tablets (1981); and Ansel, Introduction to Pharmaceutical Dosage Forms 2d Edition (1976).

In particular, pharmaceutically-acceptable carriers for systemic administration include sugars, starches, cellulose and its derivatives, malt, gelatin, talc, calcium sulfate, vegetable oils, synthetic oils, polyols, alginic acid, phosphate buffer solutions, emulsifiers, isotonic saline, and pyrogen-free water. Preferred carriers for parenteral administration include propylene glycol, ethyl oleate, pyrrolidone, ethanol, and sesame oil. Preferably, the pharmaceutically-acceptable carrier, in compositions for parenteral administration, comprises at least about 90% by weight by the total composition.

Various oral dosage forms can be used, including such solid forms as tablets, capsules, granules and bulk powders. These oral forms comprise a safe and effective amount, usually at least about 5%, and preferably from about 25% to about 50%, of the fluoroquinolonyl cephem. Tablets can be compressed, tablet triturates, enteric-coated, sugar-coated, film-coated, or multiple-compressed, containing suitable binders, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents. Preferred carriers for oral administration include gelatin, propylene glycol, cottonseed oil and sesame oil.

The compositions of this invention can also be administered topically to a subject, i.e., by the direct laying on or spreading of the composition on the epidermal or epithelial tissue of the subject. Such compositions include, for example, lotions, creams, solutions, gels and solids. These topical compositions preferably comprise a safe and effective amount, usually at least about 0.1%, and preferably from about 1% to about 5%, of the fluoroquinolonyl cephem. Suitable carriers for topical administration preferably remain in place on the skin as a continuous film, and resist being removed by perspiration or immersion in water. Generally, the carrier is organic in nature and capable of having dispersed or dissolved therein the fluoroquinolonyl cephem. The carrier may include pharmaceutically-acceptable emollients, emulsifiers, thickening agents, and solvents.

Methods of Administration:

This invention also provides a use of the fluoroquinolonyl cephem compound herein for the manufacture of a medicament in treating or preventing an infectious disorder in a human or other animal subject, by administering a safe and effective amount of a fluoroquinolonyl cephem to said subject. As used herein, an "infectious disorder" is any disorder characterized by the presence of a microbial infection. Preferred uses of this invention are for the treatment of bacterial infections. Such infectious disorders include (for example) central nervous system infections, external ear infections, infections of the middle ear (such as acute otitis media), infections of the cranial sinuses, eye infections, infections of the oral cavity (such as infections of the teeth, gums and mucosa), upper respiratory tract infections, lower respiratory tract infections, genitourinary infections, gastrointestinal infections, gynecological infections, septicemia, bone and joint infections, skin and skin structure infections, bacterial endocarditis, burns, antibacterial prophylaxis of surgery, and antibacterial prophylaxis in immunosuppressed patients (such as patients receiving cancer chemotherapy, or organ transplant patients).

The fluoroquinolonyl cephems and compositions of this invention can be administered topically or systemically. Systemic application Includes any method of introducing the fluoroquinolonyl cephem into the tissues of the body, e.g., intrathecal, epidural, intramuscular, transdermal, intravenous, intraperitoneal, subcutaneous, sublingual, rectal, and oral administration. The specific dosage of antimicrobial to be administered, as well as the duration of treatment, are mutually dependent. The dosage and treatment regimen will also depend upon such factors as the specific fluoroquinolonyl cephem used, the resistance pattern of the infecting organism to the fluoroquinolonyl cephem used, the ability of the fluoroquinolonyl cephem to reach minimum inhibitory concentrations at the site of the infection, the nature and extent of other infections (if any), the personal attributes of the subject (such as weight), compliance with the treatment regimen, and the presence and severity of any side effects of the treatment.

Typically, for a human adult (weighing approximately 70 kilograms), from about 75 mg to about 30,000 mg, more preferably from about 100 mg to about 20,000 mg more preferably from about 500 mg to about 3500 mg, of fluoroquinolonyl cephem are administered per day. Treatment regimens preferably extend from about 3 to about 56 days,

preferably from about 7 to about 28 days, in duration. Prophylactic regimens (such as avoidance of opportunistic infections in immunocompromised patients) may extend 6 months, or longer, according to good medical practice.

A preferred method of parenteral administration is through intramuscular injection. As is known and practiced in the art, all formulations for parenteral administration must be sterile. For mammals, especially humans, (assuming an approximate body weight of 70 kilograms) individual doses of from about 100 mg to about 7000 mg, preferably from about 500 mg to about 1500 mg, are acceptable.

A preferred method of systemic administration is oral. Individual doses of from about 100 mg to about 2500 mg, preferably from about 250 mg to about 1000 mg are preferred.

Topical administration can be used to deliver the fluoroquinolonyl cephem systemically, or to treat a local infection. The amounts of fluoroquinolonyl cephem to be topically administered depends upon such factors as skin sensitivity, type and location of the tissue to be treated, the composition and carrier (if any) to be administered, the particular fluoroquinolonyl cephem to be administered, as well as the particular disorder to be treated and the extent to which systemic (as distinguished from local) effects are desired.

The following non-limiting examples illustrate the compounds, compositions, processes, and uses of the present invention.

EXAMPLE I

[6R-[6a,7b]]-3-[[1-cyclopropyl-6-fluoro-1,4-dihydro-4-oxo-7-(1-piperazinyl)-3-quinolinyl]carbonyloxy]methyl]-8-oxo-7-[(2-thieny lacetyl)amino]-5-thia-1-azabicyclo[4.2.0]oct-2-ene-2-carboxylic acid, according to this invention, is made by the following general reaction sequence.

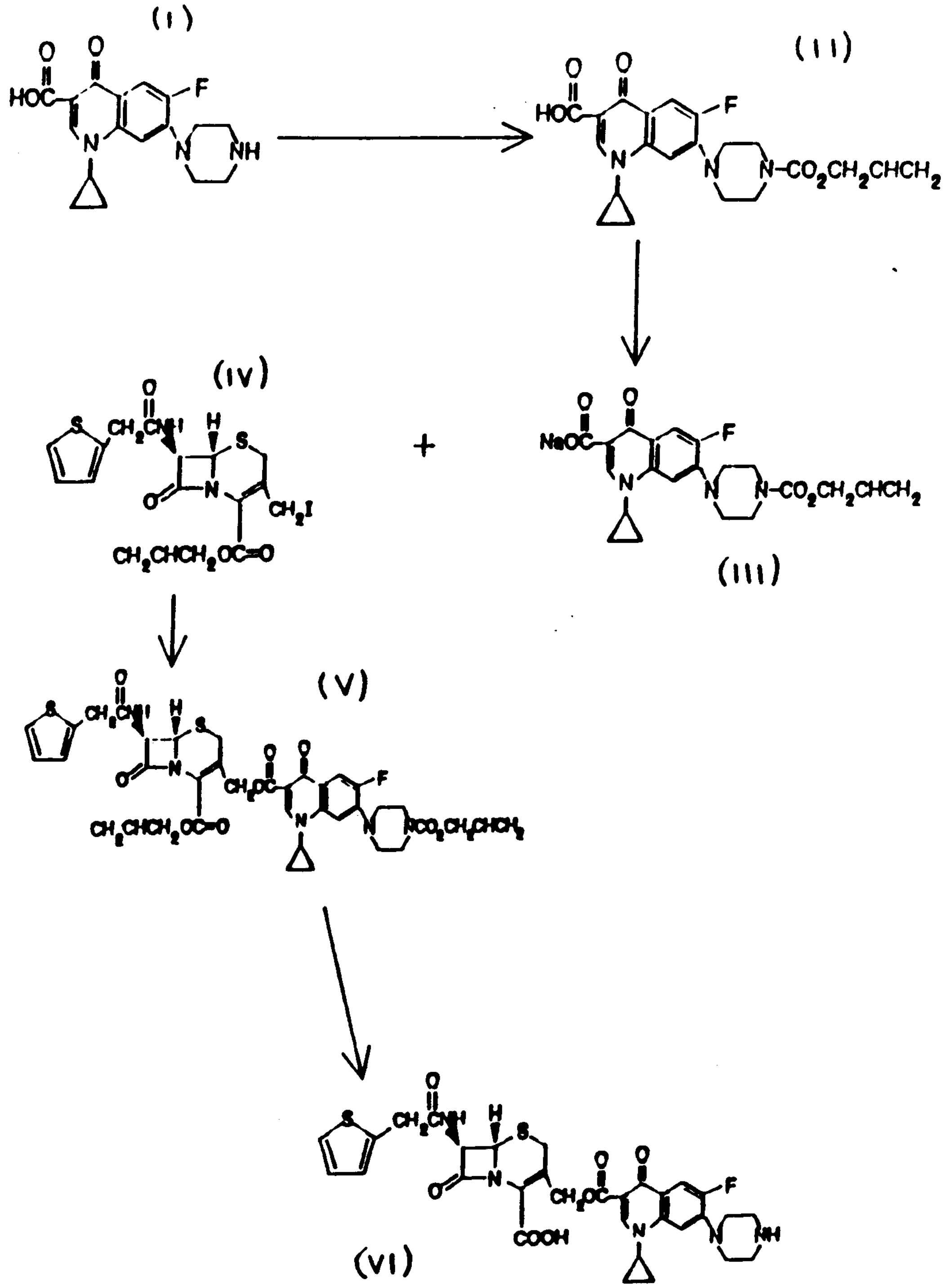

(I)
(II)
(IV)
+
(III)
(V)
(VI)

Approximately 10 g of ciprofloxacin (I) is dissolved in approximately 352 ml (milliliters) of distilled water, and cooled to approximately 0°C (32°F). The pH is then raised to approximately pH 12 by adding 1N sodium hydroxide. While stirring, approximately 88 ml of acetone is added, followed by dropwise addition of approximately 5.3 g of allylchloroformate in approximately 65.9 ml of acetone. The temperature of the solution is maintained at approximately 0°C (32°F), and the pH is maintained at approximately 12 by addition of sodium hydroxide. The reaction is stirred approximately 60 minutes.

The acetone is then evaporated from the solution. The resulting aqueous solution is twice extracted with ether. The aqueous layer is cooled, and 10% hydrochloric acid added to lower the pH to approximately pH 2.0. The solution

is then extracted three times with ethyl acetate, washed with water, dried, and evaporated to yield approximately 12 g of product (II).

This intermediate is dissolved in dichloromethane, and chilled to approximately 0°C (32°F). A solution is added dropwise, with stirring, containing approximately 1.45 g sodium hydroxide in approximately 5.0 ml methanol. Stirring is continued for approximately 60 minutes, while allowing the reaction mixture to warm to ambient temperature (approximately 21°C, 70°F). The solution is then evaporated, yielding a white solid which is triturated in ether, and collected to give approximately 11.9 g of product (III).

Separately, reactant (IV) is prepared by suspending approximately 50 g of commercially-available cephalothin, sodium salt in a mixture of DMF (dimethylformamide) (468 ml) and dioxane (375 ml). The mixture is cooled to approximately 3°C (37°F). Allyl iodide (13.2 ml, 0.144 mole) is added. The reaction is then stirred in the dark, under nitrogen, at room temperature for approximately 46 hours. This reaction mixture is poured into a mixture of saturated sodium chloride (1600 ml) and ethyl acetate (800 ml). Some solid sodium chloride is precipitated and filtered off. The layers formed are separated, and the aqueous phase extracted with ethyl acetate. The organic solutions are combined, washed with saturated sodium chloride, water, 10% sodium bicarbonate, and then water. The solution is then dried, filtered and evaporated. The residue is triturated with ether, and a solid intermediate collected by filtration.

Approximately 19.3 g of this intermediate is dissolved in dry dichloromethane (960 ml) under nitrogen. Trimethylsilyl iodide (16.3 ml, 0.0709 mole, 1.6 eq) is added. The solution is stirred in the dark, at room temperature, until no more starting material remains (approximately 1.5 hours). The solution is then cooled in ice and 10% aqueous sodium thiosulfate solution (500 ml) is added slowly, keeping the temperature at 15°C (60°F) or below. The resulting layers are separated and the organic phase is washed with 10% aqueous sodium thiosulfate and water, dried, and filtered. Acetone is added to the filtrate and the solution is filtered, and washed with 5% acetone/dichloromethane. The filtrate is then evaporated to near dryness. The residue is stirred and hexane added, precipitating a solid. The solid is collected by filtration, washed with hexane, and dried yielding reactant (IV).

Approximately 4 g of reactant (IV) is dissolved in approximately 41.8 ml of a 50% DMF/dioxane mixture, at room temperature. Approximately 3.5 g of product II is added, with stirring, at ambient temperature. Stirring is continued for approximately 90 minutes, and the solution is cooled to approximately 5°C (41°F). The mixture is then extracted with ethyl acetate, recovering the organic layer. The solution is washed five times with cooled 0.14N sodium hydroxide and with water. The solution is dried and evaporated to yield approximately 3.9 g of product (V).

Approximately 1.0 g of this intermediate is dissolved in approximately 24 ml of dry dichloromethane, containing approximately 0.128 ml of distilled water and 18 mg (milligrams) bis(triphenylphosphine)palladium chloride. Approximately 0.76 ml of tributyltin hydride is added while maintaining a temperature of approximately 21°C (70°F), forming a precipitate. After rapidly stirring for approximately 5 minutes, the precipitate is collected by filtration and dried. The precipitate is then triturated in acetone to yield approximately 328 mg of final product (VI).

EXAMPLE II

An antimicrobial composition for parenteral administration, according to this invention, is made comprising:

| Component | Amount |
|---|---|
| [6R-[6a,7b]]-3-[[1-cyclopropyl-6-fluoro-1,4-dihydro-4-oxo-7-(1-piperazinyl)-3-quinolinyl]carbonyloxy methyl]8-oxo-7-[(2-thienylacetyl)-amino]-5-thia-1-azabicyclo[4.2.0]oct-2-ene-2-carboxylic acid[1] | 100 mg/ml carrier |
| Carrier: | |
| sodium citrate buffer with (percent by weight of carrier): | |
| lecithin | 0.48% |
| carboxymethylcellulose | 0.53 |
| povidone | 0.50 |
| methyl paraben | 0.11 |
| propyl paraben | 0.011 |

1: a fluoroquinolonyl cephem, made according to Example I

The above ingredients are mixed, forming a suspension. Approximately 2.0 ml of the suspension is systemically administered, via intramuscular injection, to a human subject suffering from a lower respiratory tract infection, with Streptococcus pneumoniae present. This dosage is repeated twice daily, for approximately 14 days. After 4 days, symptoms of the disease subside, indicating that the pathogen has been substantially eradicated.

EXAMPLE III

An enteric coated antimicrobial composition for oral administration, according to this invention, is made comprising the following core tablet composition:

| Component | Amount (mg) |
|---|---|
| [6R-[6a,7b]]-3-[[1-cyclopropyl-6-fluoro-1,4-dihydro-4-oxo-7-(1-piperazinyl)-3-quinolinyl]carbonyl oxymethyl]--8-oxo-7-[(2-thienylacetyl)-amino]-5-thia-1-azabicyclo[4.2.0]oct-2-ene-2-carboxylic acid[1] | 350.0 |
| starch | 30.0 |
| magnesium stearate | 5.0 |
| microcrystalline cellulose | 100.0 |
| colloidal silicon dioxide | 2.5 |
| povidone | 12.5 |

1: a fluoroquinolonyl cephem, made according to Example I

The components are admixed into a bulk mixture. Compressed tablets are formed, using tabletting methods known in the art. The tablets are then coated with a suspension of methacrylate acid/methacrylate ester polymer in isopropanol/acetone. A human subject, having a urinary tract infection with Escherichia coli present, is orally administered two of the tablets, every 8 hours, for 14 days. Symptoms of the disease then subside, indicating substantial eradication of the pathogen.

## Claims

1. The compound

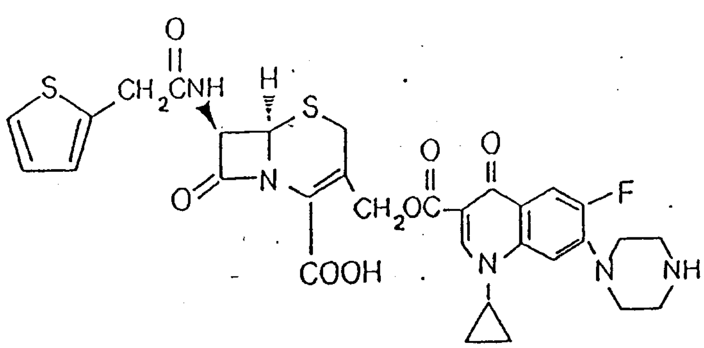

and pharmaceutically-acceptable salts [and biohydrolyzable esters thereof, and hydrates] thereof.

2. A composition for treating or preventing an infectious disorder in a human or other animal subject, comprising:

   (1) a safe and effective amount of the compound of Claim 1; and
   (2) a pharmaceutically-acceptable carrier.

3. A composition for treating or preventing an infectious disorder in a human or other animal subject, according to Claim 2, wherein said composition is suitable for parenteral administration.

4. A composition for treating or preventing an infectious disorder in a human or other animal subject, according to Claim 2, wherein said composition is suitable for oral administration.

5. Use of the compound of claim 1 for the manufacture of a medicament in treating an infectious disorder in a human or other animal subject.

**Patentansprüche**

1. Die Verbindung

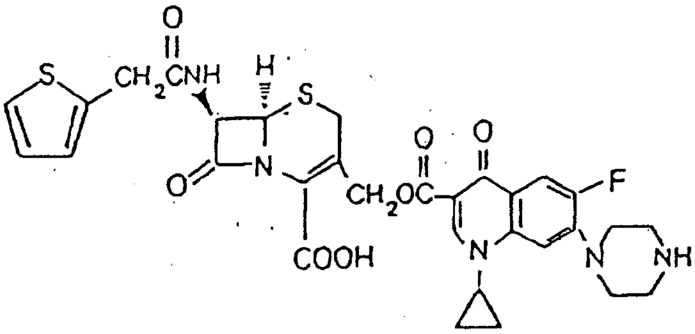

und die pharmazeutisch annehmbaren Salze hievon [und die biologisch hydrolysierbaren Ester hievon und die Hydrate hievon].

2. Zusammensetzung zur Behandlung oder Verhütung einer infektiösen Erkrankung bei einem Menschen oder einem anderen Tier, umfassend

(1) eine sichere und wirksame Menge der Verbindung nach Anspruch 1; und
(2) einen pharmazeutisch annehmbaren Träger.

3. Zusammensetzung zur Behandlung oder Verhütung einer infektiösen Erkrankung bei einem Menschen oder einem anderen Tier nach Anspruch 2, worin die genannte Zusammensetzung zur parenteralen Verabreichung geeignet ist.

4. Zusammensetzung zur Behandlung oder Verhütung einer infektiösen Erkrankung bei einem Menschen oder einem anderen Tier nach Anspruch 2, worin die genannte Zusammensetzung zur oralen Verabreichung geeignet ist.

5. Verwendung der Verbindung nach Anspruch 1 zur Herstellung eines Arzneimittels zur Behandlung einer infektiösen Erkrankung bei einem Menschen oder einem anderen Tier.

**Revendications**

1. Le composé

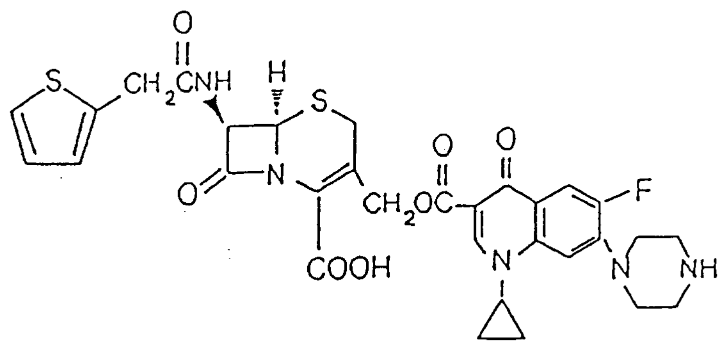

et ses sels [et esters biohydrolysables, et hydrates] pharmaceutiquement acceptables.

**2.** Composition pour traiter ou prévenir une maladie infectieuse chez un sujet humain ou animal, comprenant :

(1) une quantité efficace et dépourvue de nocivité du composé de la revendication 1 ; et
(2) un véhicule pharmaceutiquement acceptable.

**3.** Composition pour traiter ou prévenir une maladie infectieuse chez un sujet humain ou animal, selon la revendication 2, ladite composition convenant pour l'administration parentérale.

**4.** Composition pour traiter ou prévenir une maladie infectieuse chez un sujet humain ou animal, selon la revendication 2, ladite composition convenant pour l'administration orale.

**5.** Utilisation d'un composé de la revendication 1 pour la fabrication d'un médicament destiné à traiter une maladie infectieuse chez un sujet humain ou animal.